# EUROPEAN PATENT SPECIFICATION

(11) **EP 4 057 983 B1**
(45) Date of publication and mention of the grant of the patent: **03.01.2024**
(21) Application number: 20803183.1
(22) Date of filing: 12.11.2020
(51) Int. Cl.: A61K 8/04, A61K 8/27, A61K 8/31, A61K 8/37, A61K 8/92, A61Q 15/00

(54) **DEODORANT COMPOSITIONS**
DESODORIERENDE ZUSAMMENSETZUNGEN
COMPOSITIONS DÉODORANTES

(30) Priority: 15.11.2019 EP 19209486
(43) Date of publication of application: 21.09.2022
(73) Proprietor: Unilever IP Holdings B.V., 3013 AL Rotterdam (NL); Unilever Global IP Limited, Wirral, Merseyside CH62 4ZD (GB)
(72) Inventor: EVANS, Ceri, Anne, Leeds Yorkshire LS14 2AR (GB); MCWALTER, Joy, Rachel, Leeds Yorkshire LS14 2AR (GB)
(74) Representative: Whaley, Christopher
(86) International application number: PCT/EP2020/081866
(87) International publication number: WO 2021/094429

(56) References cited:
- EP-A1- 2 604 249
- EP-A1- 2 949 312
- WO-A1-2018/087147
- WO-A1-2018/087148
- US-A1- 2010 189 665

## Description

### Field of Invention

The present invention relates to deodorant aerosol compositions and their dispensing.

### Background

Deodorant aerosol compositions have been marketed for many years. Typically, such compositions comprise a deodorant material, a carrier fluid and a propellant. A variety of carrier fluids have been disclosed in the prior art, some of which are relatively hydrophilic, such as ethanol, others being more hydrophobic.

Hydrophobic carrier fluids used in the past have included silicone fluids, such cyclomethicones, and hydrocarbon fluids. Hydrocarbon fluids disclosed in the prior art include mixtures of C11 and C13 alkanes as described in WO 08/15560 A2 (Cognis, 2008) and other members of the same extended patent family. EP2949312 relates to silicone-free, aerosol deodorant antiperspirant compositions.

Deodorant actives used in past have include both hydrophilic and hydrophobic materials.

One of the more hydrophobic groups of deodorant actives disclosed are zinc carboxylates, as disclosed in WO 18/087147 and WO 18/087148 (both 2018 and both by Givaudan).

### Summary of Invention

It is an object of the present invention to provide a deodorant aerosol composition that does not require cyclic silicones, in particular cyclopentasiloxane, otherwise known as D5.

It is a further object of the present invention to provide a deodorant aerosol composition that has good sensory properties.

It is a further object of the present invention to provide a method of manufacture of a deodorant aerosol composition that has good ease of process and results in an effective product.

It is a further object of the present invention to provide a deodorant aerosol composition that has good stability.

It is a further object of the present invention to provide a deodorant aerosol composition that has good dispensing, i.e. a composition that does not give significant valve and/or spray channel blockage.

In a first aspect of the invention, there is provided a deodorant aerosol composition comprising (i) a deodorant material consisting of a mixture of zinc neodecanoate, a perfumery-compatible solvent oil and at least one fragrance material, (ii) a a hydrocarbon carrier fluid which is liquid at 20°C, and (iii) a propellant, wherein the hydrocarbon carrier fluid is saturated and has at least ten carbon atoms, wherein the propellant comprises a liquified hydrocarbon which is gaseous at 20°C and atmospheric pressure; and wherein the composition does not comprise cyclic liquid polyorganosiloxanes.

In a second aspect of the invention, there is provided the use of a deodorant aerosol composition according to the first aspect of the invention to deliver deodorancy benefits to the surface of the human body.

In a fourth aspect of the present invention, there is provided a deodorant aerosol composition according to the first aspect of the invention and aerosol hardware comprising a can capable of withstanding elevated pressures, a spray channel terminating with a spray orifice and a valve which, when actuated, serves to release the composition from the can and into the spray channel.

### Detailed Description

Herein, features expressed as "preferred" with regard to a particular aspect of the invention should be understood to be preferred with regard to each aspect of the invention (likewise, features expressed as "more preferred" or "most preferred").

Herein, preferred features of the invention are particularly preferred when used in combination with other preferred features.

Herein, all percentages, ratios and amounts are by weight, unless otherwise indicated.

Herein, all percentages, ratios and amounts are by weight, .

Herein, all percentages, ratios and amounts are by weight of the total composition, unless otherwise indicated.

Herein, all percentages, ratios and amounts are to be understood to be modified by the word "about" where appropriate.

Herein, the word "comprising" is intended to mean "including" but not necessarily "consisting of", i.e., it is non-exhaustive.

Herein, cosmetic methods are to be understood as a non-therapeutic in nature.

Herein, references to an amount of a compound or an active refer to the total amount of compounds or actives of the type indicated.

Herein, "application" and "applied" relate to application to the surface of the human body, in particular the underarm regions.

Herein, compositions of the invention comprise a "base" composition and a propellant. The base composition or "base" consists of all components of the total composition excluding the propellant.

The base: propellant ratio is preferably from 50: 50 to 95: 5, more preferably from 50: 50 to 93: 7 and most preferably from 70: 30 to 91: 9.

The inventors have discovered that superior deodorant aerosol compositions may be formed by incorporating the components as described herein. The superiority may take the form of enhanced deodorancy performance, stability, dispensing and/or enhanced sensory properties. In addition, benefits may be found in the odour stability of the compositions.

Herein, "odour stability" refers to a composition's ability to maintain its odour; in particular, its ability not to develop malodour. This may be considered an aspect of hedonic stability.

One or more of the enhancements referred to in the paragraph immediately above may be achieved by having a composition which is a homogeneous solution and preferably a true solution. Factors favouring such homogeneity are that both zinc neodecanoate and the hydrocarbon carrier fluid have saturated hydrocarbyl chains and have at least ten carbon atoms in each hydrocarbon or carboxylate chain present therein. The homogeneity is also favoured when the propellant comprises a liquified hydrocarbon, particularly when the propellant consists solely of liquified hydrocarbon.

Herein, homogeneous refers to a composition in which the components are in the same phase, this phase being a liquid phase. Homogeneous solutions as described herein may be true solutions or colloidal solutions or emulsions. Excluded from this definition are dispersions or solid particulates suspended in a liquid phase.

Benefits for compositions which are homogeneous solutions are good stability and good dispensing. The stability may be manifested by a failure to gel or phase separate. The good dispensing may be manifested by ease of passage of the composition through the narrow orifices within nozzles typically used to spray aerosol compositions. Blockage of nozzles can be a problem with some other compositions.

The deodorant material consists of a mixture of zinc neodecanoate, a perfumery-compatible solvent oil and at least one fragrance material.

Zinc neodecanoate may be represented by the formula: [C₉H₂₀-CO.O]₂Zn.

The deodorant material is preferably present in the composition at from 0.01 to 10%, more preferably at from 0.1% to 5% and most preferably at from 0.5% to 3%. The zinc neodecanoate typically comprises from 50 to 95% of the deodorant material and more typically from 70 to 90% of the deodorant material.

Herein, a "perfumery-compatible solvent oil" is an oil having good compatibility with one or more perfume or fragrance oils. The good combability is usually evidenced by the solvent oil being able to dissolve one or more fragrance oils. In preferred embodiments, the perfumery-compatible solvent oil is also able to dissolve the zinc neodecanoate.

The perfumery-compatible solvent oil typically comprises from 5 to 50% of the deodorant material and more typically from 10 to 30% of the deodorant material.

Herein, "oils" are water-immiscible liquids having a melting point above 25°C.

Preferred perfumery-compatible solvent oils are ester oils. Examples of suitable ester oils include oils containing one or two alkyl groups of 12 to 24 carbon atoms length, including isopropyl myristate, isopropyl palmitate and myristyl palmitate. Other non-volatile ester oils include alkyl or aryl benzoates such C₁₂₋₁₅ alkyl benzoate.

Herein, the carrier fluid refers to components of compositions of the invention other than the deodorant material and the propellant that are "fluid", i.e. capable of flow at 20°C and atmospheric pressure.

In preferred embodiments, the carrier fluid is liquid at 25°C or greater and more preferably it is liquid at 20°C or greater, both assessments being at atmospheric pressure.

The content of unsaturated hydrocarbons in the composition is preferably kept low. The ratio of unsaturated hydrocarbons to saturated hydrocarbons is preferably less than 1: 5, more preferably less than 1: 10 and most preferably less than 1: 20.

Herein, unsaturated hydrocarbons comprise one or more carbon-carbon double or triple bonds and saturated hydrocarbons are free from carbon-carbon double or triple bonds. Preferred compositions of the invention may comprise an ester or ether oil. Such oils are not to be considered hydrocarbon oils (*vide infra*). Such oils may contribute to the sensory properties of the composition. They may also contribute to the stability and good dispensing of the formulation.

Ester and/or ether oils are incorporated into the composition at a preferred level of from 1 to 10%, more preferably from 2 to 8% and most preferably at from 3 to 6%.

The compositions do not comprise cyclic liquid polyorganosiloxanes. More preferred compositions do not comprise liquid polyorganosiloxanes whatsoever.

Particularly suitable additional components may include alkyl ether oils having a boiling point of above 100°C and especially above 150°C, including polyalkyleneglycol alkyl ethers. Such ethers desirably comprise between 10 and 20 ethylene glycol or propylene glycol units and the alkyl group commonly contains from 4 to 20 carbon atoms. The preferred ether oils include polypropylene glycol alkyl ethers such as PPG-14-butylether and PPG-15-stearyl ether.

Suitable additional components can include one or more triglyceride oils. The triglyceride oils commonly comprise the alkyl residues of aliphatic C₇ to C₂₀ alcohols, the total number of carbon atoms being selected in conjunction with the extent of olefinic unsaturation and/or branching to enable the triglyceride to be liquid at 20°C. One example is jojoba oil. Particularly preferred in the triglyceride oil are carboxylate groups that are linear C₁₈ groups having one, two or three olefinic degrees of unsaturation, two or three being optionally conjugated, many of which are extractable from plants (or their synthetic analogues), including triglycerides of oleic acid, linoleic acid, conjugated linoleic acids, linolenic acid, petroselenic acid, ricinoleic acid, linolenelaidic acid, trans 7-octadecenoic acid, parinaric acid, pinolenic acid, punicic acid, petroselenic acid and stearidonic acid.

Suitable oils that can be included are those derived from unsaturated C₁₈ acids, including coriander seed oil, impatiens balsimina seed oil, parinarium laurinarium kernel fat oil, sabastiana brasilinensis seed oil, dehydrated castor seed oil, borage seed oil, evening primrose oil, aquilegia vulgaris oil, sunflower (seed) oil and safflower oil. Other suitable oils are obtainable from hemp, and maize corn oil. An especially preferred oil by virtue of its characteristics is sunflower (seed) oil.

Further suitable additional components, that can also be emollient oils, comprise alkyl or alkyl-aryl ester oils having a boiling point of above 150°C (and a melting point of below 20°C). Such ester oils include oils containing one or two alkyl groups of 12 to 24 carbon atoms length, including isopropyl myristate, isopropyl palmitate and myristyl palmitate. Other non-volatile ester oils include alkyl or aryl benzoates such C₁₂₋₁₅ alkyl benzoate.

A further class of additional components are non-volatile dimethicones, often comprising phenyl or diphenylene substitution, for example Dow Corning 200 350cps or Dow Corning 556.

Herein, the term "hydrocarbon" refers to a material consisting solely of carbon and hydrogen atoms and the term "saturated" refers to a material having no degree of unsaturation.

The saturated nature of the hydrocarbon carrier fluid may contribute to the odour stability of the composition, particularly in conjunction with the saturated nature of the zinc neodecanoate.

The preferred features of the hydrocarbon referred to in the following paragraphs may each aid the homogeneity of the overall compositions and the other benefits of the compositions of the invention.

The saturated hydrocarbon carrier fluid is liquid at 20°C and has at least ten carbon atoms. Even more preferably, the saturated hydrocarbon carrier fluid has from 10 to 14 carbon atoms. The saturated hydrocarbon carrier fluid is preferably linear and non-cyclic, whether it comprises at least 10 carbon atoms or from 10 to 14 carbon atoms. In certain most preferred embodiments, the saturated hydrocarbon carrier fluid is linear, non-cyclic and has from 11 to 13 carbon atoms. A blend of undecane and tridecane may be advantageously employed, particularly at a ratio of from 50: 50 to 80: 20. Such materials are available from BASF under the tradename Cetiol Ultimate.

When a mixture of saturated hydrocarbons is present, it is preferred that said mixture of saturated hydrocarbons is liquid at 25°C or greater and more preferably it is liquid at 20°C or greater.

When a mixture of saturated hydrocarbons is present, it is preferred that all the saturated hydrocarbons have at least 8 carbon atoms and more preferably at least 10. Even more preferably, the saturated hydrocarbons all have from 10 to 14 carbon atoms. In certain most preferred embodiments, the saturated hydrocarbons are all linear, non-cyclic and have from 11 to 13 carbon atoms.

The hydrocarbon carrier fluid is typically present in the base composition at from 10 to 70%, preferably at from 10 to 50% and more preferably at from 15 to 50%.

The hydrocarbon carrier fluid is typically present in compositions of the invention at from 1 to 10%, preferably at from 1 to 5% of the full composition.

The propellant comprises liquefied hydrocarbon gases, and especially C₃ to C₆ hydrocarbons, including propane, isopropane, butane, isobutane, pentane and isopentane and mixtures of two or more thereof. Preferred propellants are isobutane, isobutane/isopropane, isobutane/propane and mixtures of isopropane, isobutane and butane.

Herein, a liquified hydrocarbon gas should be understood to be a material which is gaseous at 20°C and atmospheric pressure.

In especially preferred embodiments, the propellant consists solely of liquified hydrocarbon, as detailed in any of the selections detailed in the paragraph immediately above, for example.

The propellant is incorporated into the composition preferably at a level of from 50 to 95%, more preferably from 50 to 90% and most preferably from 70 to 90%.

Fragrance is a preferred addition component of compositions of the invention. Encapsulated fragrances are particularly preferred. The total fragrance content in the base of compositions of the invention is commonly up to 3%, advantageously is at least 0.5% and particularly from 0.8% to 2%.

Compositions of the invention may comprise conventional antiperspirant actives that are astringent aluminium salts. However, preferred compositions of the invention do not comprise such salts as these can be detrimental to the homogeneity of the composition and may lead to dispensing and/or sensory negatives.

Preferred compositions of the invention comprise an antioxidant, such as BHT (butylated hydroxytoluene) or dilauryl thiodipropionate, typically at a level of from 0.001 to 0.5%.

The composition is preferably anhydrous, i.e. a composition containing less than 2% by weight of water, or even less than 0.5% of water, especially water-free.

Compositions of the invention may be prepared by conventional manufacturing methods. In preferred embodiments, all the components of the base are mixed with stirring to give a homogenous solution and this solution is then gassed with the propellant.

Compositions of the invention may be used in conjunction with conventional aerosol hardware. Typically, the composition is stored in a pressurised can and is dispensed through a spray channel by actuation of a valve between the pressurised can and the spray channel. The spray channel generally terminates with a spray orifice through which the compositions exits with atomisation to form a spray cloud.

The invention is illustrated in greater detail by means of the examples described below.

### Examples

The Examples detailed in Table 1 were prepared by methods known in the art. All of the Examples prepared were homogenous solutions.

Examples 1 and 2 were assessed as to their ability to reduce malodour development on the surface of the human body, specifically the underarm regions. Both products performed well.

Examples 1 and 3 (lacking fragrance) were assessed as to their odour stability. Samples were placed on storage at 45°C for at least 12 weeks. The sample performed well, generating no significant malodour over the course of the test.

Aerosol technical testing was performed on Example 4 using standard aerosol hardware. An acceptable spray quality was achieved, indicating good dispensing and no valve blockage.

**Table 1**

| Ingredient | Example | | | | |
|---|---|---|---|---|---|
| | 1 | 2 | 3 | 4 | 5 |
| **Ingredients** | | | | | |
| Undecane and Tridecane* | 4.55 | 3.625 | 3.625 | 2.600 | 2.94 |
| PPG-14 butyl ether | 1.75 | 0.875 | 0.875 | 2.700 | 2.95 |
| C12-15 Alkyl benzoate | 1.00 | 2.30 | 2.30 | 1.00 | 1.00 |
| Dilauryl thiodipropionate | 0.10 | 0.10 | 0.10 | 0 | 0 |
| Butylated hydroxytoluene | 0 | 0 | 0 | 0.10 | 0 |
| Octyldodecanol | 0.10 | 0.10 | 0.10 | 0.10 | 0.10 |
| Sunflower seed oil | 0.50 | 0.50 | 0.50 | 0.50 | 0.5 |
| Deodorant material: | 1.00 | 1.50 | 1.50 | 1.00 | 1.00 |
| zinc neodecanoate + isopropyl myristate at 4: 1 and including | | | | | |
| fragrance at: | 0.01 | 0.015 | 0 | 0.01 | 0.01 |
| Additional fragrance | 0 | 0 | 0 | 1.00 | 1.00 |
| Propellant (propane, butane, isobutane) | 91 | 91 | 91 | 91 | 90 |

| | | | | | |
|---|---|---|---|---|---|
| * at an undecane to tridecane ratio of approximately 73: 27. | | | | | |

Further examples as detailed in Table 2 were also prepared by methods known in the art.

**Table 2**

| Ingredient | Example | | | | |
|---|---|---|---|---|---|
| | 6 | 7 | 8 | 9 | 10 |
| **Ingredients** | | | | | |
| Undecane and Tridecane* | 2.94 | 2.59 | 3.54 | 9.08 | 9.1 |
| PPG-14 butyl ether | 2.95 | 2.95 | 1.75 | 3.50 | 3.50 |
| C12-15 Alkyl benzoate | 1.00 | 1.00 | 1.00 | 2.00 | 2.00 |
| Dilauryl thiodipropionate | 0 | 0 | 0.10 | 0.20 | 0.20 |
| Butylated hydroxytoluene | 0 | 0.10 | 0 | 0 | 0 |
| Octyldodecanol | 0.10 | 0.10 | 0.10 | 0.20 | 0.20 |
| Sunflower seed oil | 0.50 | 0.50 | 0.50 | 1.00 | 1.00 |
| Deodorant material: | 1.00 | 1.00 | 1.00 | 2.00 | 2.00 |
| zinc neodecanoate + isopropyl myristate at 4: 1 and including | | | | | |
| fragrance at: | 0.01 | 0.01 | 0.01 | 0.02 | 0 |
| Additional fragrance | 1.50 | 1.75 | 1.00 | 0 | 0 |
| Propellant (propane, butane, isobutane) | To 100 | To 100 | To 100 | To 100 | To 100 |

| | | | | | |
|---|---|---|---|---|---|
| * at an undecane to tridecane ratio of approximately 73: 27. | | | | | |

Examples 8, 9 and 10 were assessed as to their odour stability. Samples were placed on storage at 45°C for at least 12 weeks. Each of the examples performed well, generating no significant malodour over the course of the test.

## Claims

1. A deodorant aerosol composition comprising (i) a deodorant material consisting of a mixture of zinc neodecanoate, a perfumery-compatible solvent oil and at least one fragrance material, (ii) a hydrocarbon carrier fluid which is liquid at 20°C, and (iii) a propellant, wherein the hydrocarbon carrier fluid is saturated and has at least ten carbon atoms; wherein the propellant comprises a liquified hydrocarbon which is gaseous at 20°C and atmospheric pressure; and wherein the composition does not comprise cyclic liquid polyorganosiloxanes.

2. A composition according to claim 1, wherein the propellant consists solely of liquified hydrocarbon.

3. A composition according to claim 1 or claim 2, wherein the propellant comprises one or more of propane, butane or isobutane.

4. A composition according to any of the preceding claims comprising an ester or ether oil.

5. A composition according to claim 4, wherein the ester or ether oil is a triglyceride oil.

6. A composition according to any of the preceding claims, comprising an encapsulated fragrance.

7. A composition according to any of the preceding claims, wherein the saturated hydrocarbon in the carrier fluid has from 10 to 14 carbon atoms.

8. A composition according to any of the preceding claims, wherein the perfumery-compatible solvent oil is an ester oil.

9. A composition according to any of the preceding claims, wherein the composition is anhydrous.

10. A composition according to any of the preceding claims, wherein the base to propellant ratio is from 50: 50 to 95: 5 by weight, the base consisting of all components of the total composition apart from the propellant.

11. A composition according to any of the preceding claims, wherein the composition is a homogeneous solution.

12. Use of a composition according to any of the preceding claims for the delivery of antiperspirancy and sensory benefits to the surface of the human body.

13. A composition according to any of claims 1 to 11 and aerosol hardware comprising a can capable of withstanding elevated pressures, a spray channel terminating with a spray orifice and a valve which, when actuated, serves to release the composition from the can and into the spray channel.

## Patentansprüche

1. Desodorierende Aerosolzusammensetzung, umfassend (i) ein desodorierendes Material, bestehend aus einer Mischung aus Zinkneodecanoat, einem parfümverträglichen Lösungsmittelöl und mindestens einem Duftstoffmaterial, (ii) eine Kohlenwasserstoff-Trägerflüssigkeit, die bei 20°C flüssig ist, und (iii) ein Treibmittel, wobei die Kohlenwasserstoff-Trägerflüssigkeit gesättigt ist und mindestens zehn Kohlenstoffatome aufweist; wobei das Treibmittel einen verflüssigten Kohlenwasserstoff umfasst, der bei 20°C und Atmosphärendruck gasförmig ist; und wobei die Zusammensetzung keine flüssigen cyclischen Polyorganosiloxane umfasst.

2. Zusammensetzung nach Anspruch 1, wobei das Treibmittel ausschließlich aus verflüssigtem Kohlenwasserstoff besteht.

3. Zusammensetzung nach Anspruch 1 oder Anspruch 2, wobei das Treibmittel eines oder mehrere von Propan, Butan oder Isobutan umfasst.

4. Zusammensetzung nach einem der vorhergehenden Ansprüche, umfassend ein Ester- oder Etheröl.

5. Zusammensetzung nach Anspruch 4, wobei das Ester- oder Etheröl ein Triglyceridöl ist.

6. Zusammensetzung nach einem der vorhergehenden Ansprüche, umfassend einen eingekapselten Duftstoff.

7. Zusammensetzung nach einem der vorhergehenden Ansprüche, wobei der gesättigte Kohlenwasserstoff in der Trägerflüssigkeit 10 bis 14 Kohlenstoffatome aufweist.

8. Zusammensetzung nach einem der vorhergehenden Ansprüche, wobei das parfümverträgliche Lösungsmittelöl ein Esteröl ist.

9. Zusammensetzung nach einem der vorhergehenden Ansprüche, wobei die Zusammensetzung wasserfrei ist.

10. Zusammensetzung nach einem der vorhergehenden Ansprüche, wobei das Verhältnis von Basis zu Treibmittel gewichtsmäßig 50:50 bis 95:5 beträgt, wobei die Basis aus allen Komponenten der Gesamtzusammensetzung, abgesehen vom Treibmittel, besteht.

11. Zusammensetzung nach einem der vorhergehenden Ansprüche, wobei die Zusammensetzung eine homogene Lösung ist.

12. Verwendung einer Zusammensetzung nach einem der vorhergehenden Ansprüche zur Bereitstellung schweißhemmender und sensorischer Wirkungen auf der Oberfläche des menschlichen Körpers.

13. Zusammensetzung nach einem der Ansprüche 1 bis 11 und Aerosolausrüstung, umfassend eine Dose, die erhöhten Drücken standhalten kann, einen Sprühkanal, der mit einer Sprühöffnung endet, und ein Ventil, das bei Betätigung dazu dient, die Zusammensetzung aus der Dose und in den Sprühkanal freizugeben.

## Revendications

1. Composition d'aérosol de déodorant comprenant (i) un matériau de déodorant consistant en un mélange de néodécanoate de zinc, d'une huile de solvant compatible avec la parfumerie et d'au moins un matériau de parfum, (ii) un fluide de support hydrocarboné qui est liquide à 20°C, et (iii) un gaz propulseur, dans laquelle le fluide de support hydrocarboné est saturé et présente au moins dix atomes de carbone ; dans laquelle le gaz propulseur comprend un hydrocarbure liquéfié qui est gazeux à 20°C et pression atmosphérique ; et dans laquelle la composition ne comprend pas de polyorganosiloxanes liquides cycliques.

2. Composition selon la revendication 1, dans laquelle le gaz propulseur consiste uniquement en hydrocarbure liquéfié.

3. Composition selon la revendication 1 ou revendication 2, dans laquelle le gaz propulseur comprend un ou plusieurs parmi le propane, butane ou isobutane.

4. Composition selon l'une quelconque des revendications précédentes comprenant une huile d'ester ou d'éther.

5. Composition selon la revendication 4, dans laquelle l'huile d'ester ou d'éther est une huile de triglycéride.

6. Composition selon l'une quelconque des revendications précédentes, comprenant un parfum encapsulé.

7. Composition selon l'une quelconque des revendications précédentes, dans laquelle l'hydrocarbure saturé dans le fluide de support présente de 10 à 14 atomes de carbone.

8. Composition selon l'une quelconque des revendications précédentes, dans laquelle l'huile de solvant compatible avec la parfumerie est une huile d'ester.

9. Composition selon l'une quelconque des revendications précédentes, dans laquelle la composition est anhydre.

10. Composition selon l'une quelconque des revendications précédentes, dans laquelle le rapport base à gaz propulseur est de 50:50 à 95:5 en masse, la base consistant en tous les constituants de la composition totale à part le gaz propulseur.

11. Composition selon l'une quelconque des revendications précédentes, dans laquelle la composition est une solution homogène.

12. Utilisation d'une composition selon l'une quelconque des revendications précédentes pour la délivrance de bénéfices antiperspirants et sensoriels sur la surface du corps humain.

13. Composition selon l'une quelconque des revendications 1 à 11 et matériel d'aérosol comprenant un récipient capable de résister aux pressions élevées, un canal de pulvérisation se terminant avec un orifice de pulvérisation et une valve qui, lorsque actionnée, sert à libérer la composition à partir du récipient et dans le canal de pulvérisation.
